# EUROPEAN PATENT APPLICATION

(11) **EP 3 936 607 A1**
(43) Date of publication of application: **12.01.2022**
(21) Application number: 20770800.9
(22) Date of filing: 04.03.2020
(51) Int. Cl.: C12N 5/02, C12N 5/071, C12N 5/074, C12N 5/075, C12N 5/076, C12N 5/077, C12N 5/078, C12N 5/0783, C12N 5/0784, C12N 5/0789, C12N 5/0793, C12N 5/10, A61K 35/12

(54) **MASS CULTURE OF PLURIPOTENT STEM CELLS**

(30) Priority: 08.03.2019 JP 2019042797
(71) Applicant: Kaneka Corporation, Osaka-shi, Osaka 530-8288 (JP)
(72) Inventor: MATSUTA, Hirotoshi, Kobe-shi, Hyogo 650-0047 (JP); UEDA, Yasuyoshi, Kobe-shi, Hyogo 650-0047 (JP)
(74) Representative: Hoffmann Eitle
(86) International application number: PCT/JP2020/009268
(87) International publication number: WO 2020/184350

(57) **Abstract**

A production method for pluripotent stem cells is provided, the method including the following (a) and (b): (a) a process filling a culture container with a liquid medium and thereafter raising, the temperature of the liquid medium in the culture container to the temperature at which pluripotent stem cells can proliferate; and (b) a process seeding pluripotent stem cells in the liquid medium in the culture container and culturing the pluripotent stem cells in suspension.

## Description

### [Technical Field]

The present invention relates to a method for mass culture of pluripotent stem cells on a commercial scale, and a method for producing somatic cells from the pluripotent stem cells obtained by the above method.

Priority is claimed on Japanese Patent Application No. 2019-42797, filed March 8, 2019, the content of which is incorporated herein by reference.

### [Background Art]

Pluripotent stem cells such as ES cells and iPS cells have an ability to grow indefinitely and multilineage potential to differentiate into various cells. The possibility of practical use of fundamental treatments for intractable diseases and lifestyle-related diseases utilizing the cellular properties of pluripotent stem cells is increasing due to accomplishments of recent research.

For example, it has already become possible to induce differentiation, from pluripotent stem cells, into cardiac muscle cells, skeletal muscle cells, nerve cells, megakaryocytes, hematopoietic stem cells, airway epithelial cells, germ cells, dendritic cells, eosinophils, mast cells, cartilage cells, T cells, erythropoietin-producing cells, intestinal epithelium, pancreatic cells, hepatocytes, alveolar epithelial cells, and the like.

Meanwhile, regenerative medicine using pluripotent stem cells still has problems in terms of practical use. An example of the problems is Productivity of pluripotent stem cells. In general, regenerative medicine requires a large amount of pluripotent stem cells. For example, 20 billion pluripotent stem cells are required to treat the liver. Therefore, pluripotent stem cells are required to be produced efficiently in large amounts, but a general plate culture requires 10⁶ cm² or larger for a culture area, which is equivalent to 20000 pieces of a 10 cm dish that is a general culture dish. Plate culture as described above is extremely difficult to be scaled up, and is not realistic because securing of a huge area of a production facility is also required.

In order to solve these problems, in recent years, suspension culture in which cells are three-dimensionally cultured while suspending them in a medium has been reported.

For example, Patent Document 1 discloses a technique for producing aggregates of cells by culturing while performing gyratory culture of cells in a medium.

Furthermore, Patent Document 2 discloses a technique in which suspension culture is performed so that an average diameter of cell masses is 200 µm or more and 300 µm or less by adding, as means for preventing adhesion between the cell masses, a water-soluble polymer into a medium to increase viscosity.

### [Citation List]

### [Patent Documents]

[Patent Document 1]
   Japanese Unexamined Patent Application, First Publication No. 2003-304866
[Patent Document 2]
   PCT International Publication No. WO2013/077423

### [Summary of Invention]

### [Technical Problem]

In order to perform mass culture of pluripotent stem cells, the present inventors repeated an operation of heating a commercially available cell culture medium and injecting the medium heated to around 37°C into a culture container heated to around 37°C, until the volume reached a volume enabling suspension culture. Next, as a result of seeding pluripotent stem cells in the culture container to perform suspension culture while stirring the cells, we found a problem that growth of the pluripotent stem cells was lower as compared to growth of pluripotent stem cells during plate culture. Furthermore, with analyzing the undifferentiated state of the pluripotent stem cells obtained by the suspension culture, we also found a problem that pluripotent stem cells deviating from the undifferentiated state increased.

### [Solution to Problem]

As a result of diligent studies to solve the above problems, the present inventors found that, by repeating the operation of injecting a heated medium into a heated culture container, components in the medium injected into the culture container from an initial stage to a middle stage are destabilized, and the influence of this destabilization causes a phenomenon in which growth of pluripotent stem cells is lowered. Furthermore, it was found that this phenomenon induces the deviation of pluripotent stem cells from the undifferentiated state. Therefore, the present inventors performed suspension culture by raising a temperature of a liquid medium in a culture container to around 37°C after injecting the medium into the culture container until the volume reached a volume enabling suspension culture, and thereafter seeding pluripotent stem cells in this culture container. As a result, the present inventors obtained surprising findings that, by performing the above-described culture method, growth of pluripotent stem cells is improved, and the undifferentiated state is maintained, and therefore completed the present invention.

Specifically, the present invention includes the following inventions.
(1) A production method for pluripotent stem cells, the method including the following (a) and (b):
   (a) a process of filling a culture container with a liquid medium and thereafter raising, the temperature of the liquid medium in the culture container to the temperature at which pluripotent stem cells can proliferate and
   (b) a process of seeding pluripotent stem cells in the liquid medium in the culture container and culturing the pluripotent stem cells in suspension.
(2) The production method according to (1), in which in the process (a), the temperature of the liquid medium is raised by at least 20°C.
(3) The production method according to (1) or (2), in which in the process (a), the temperature of the liquid medium in the culture container before raising the temperature is -20°C or higher and 10°C or lower.
(4) The production method according to any one of (1) to (3), in which in the process (a), the temperature at which the pluripotent stem cells grow is 30°C or higher and 40°C or lower.
(5) The production method according to any one of (1) to (4), in which in the process (a), the temperature of the liquid medium is raised with stirring the liquid medium in the culture container.
(6) The production method according to any one of (1) to (5), in which a growth factor is not added in the process (a).
(7) The production method according to any one of (1) to (6), in which a growth factor is added in the process (b).
(8) The production method according to any one of (1) to (7), wherein the liquid medium contains at least one from the group consisting of L-ascorbic acid, insulin, transferrin, selenium, and sodium hydrogen carbonate.
(9) The production method according to any one of (1) to (8), in which the pluripotent stem cells are embryonic stem cells or induced pluripotent stem cells (iPS cells).
(10) The production method according to (6) or (7), in which the growth factor is at least one selected from the group consisting of FGF2 and TGF-β1.
(11) A production method for somatic cells, the method including the following (a) to (c):
   (a) a process of filling a culture container with a liquid medium and thereafter raising, the temperature of the liquid medium in the culture container to the temperature at which pluripotent stem cells can proliferate;
   (b) a process of seeding pluripotent stem cells in the liquid medium in the culture container and culturing the pluripotent stem cells in suspension; and
   (c) a process of culturing the pluripotent stem cells obtained in the process (b) in the presence of a differentiation-inducing factor to induce differentiation.
(12) The production method according to (11), in which the temperature of the liquid medium is raised by at least 20°C in the process (a).
(13) The production method according to (11) or (12), in which the temperature of the liquid medium in the culture container before raising the temperature is -20°C or higher and 10°C or lower in the process (a).
(14) The production method according to any one of (11) to (13), in which the temperature at which the pluripotent stem cells grow is 30°C or higher and 40°C or lower in the process (a).
(15) The production method according to any one of (11) to (14), in which the temperature of the liquid medium is raised while stirring the liquid medium in the culture container in the process (a).
(16) The production method according to any one of (11) to (15), in which a growth factor is not added in in the process (a).
(17) The production method according to any one of (11) to (16), in which a growth factor is added in the process (b).
(18) The production method according to any one of (11) to (17), in which the somatic cells are at least one selected from the group consisting of cardiac muscle cells, skeletal muscle cells, nerve cells, megakaryocytes, hematopoietic stem cells, airway epithelial cells, germ cells, dendritic cells, eosinophils, mast cells, cartilage cells, T cells, erythropoietin-producing cells, intestinal epithelium, pancreatic cells, hepatocytes, alveolar epithelial cells, and kidney cells.
(19) A pharmaceutical product composition including the somatic cells obtained by the production method according to any one of (11) to (18) as an active ingredient.
(20) The pharmaceutical product composition according to (19), in which the pharmaceutical product composition is a liquid medication for injection.
(21) The pharmaceutical product composition according to (19), in which the pharmaceutical product composition is a formulation for transplantation having a cell mass-like structure or a sheet-like structure.
(22) The production method according to any one of (1) to (18), in which the temperature of the liquid medium when filling the culture container with the liquid medium is higher than 0°C and 15°C or lower in the process (a).

### [Advantageous Effects of Invention]

According to the production method for pluripotent stem cells of the present invention, a large amount of pluripotent stem cells can be produced in a state where growth of the pluripotent stem cells is improved with maintaining the undifferentiated state of the pluripotent stem cells.

According to the production method for somatic cells of the present invention, somatic cells can be produced efficiently and in large amounts.

According to the pharmaceutical product composition of the present invention, by using it, it is possible to treat diseases such as intractable diseases and lifestyle-related diseases.

### [Description of Embodiments]

Hereinafter, embodiments of the present invention will be specifically described, but the following descriptions are for facilitating the understanding of the present invention, and the scope of the present invention is not limited to the following embodiments. The scope of the present invention also includes other embodiments in which configurations of the following embodiments are appropriately replaced by those skilled in the art.

In the present invention, a "cell" can be a cell having adhesiveness (adherent cell). The adherent cell can be an animal-derived cell or the like; can be preferably a mammalian animal-derived cell or the like; can be more preferably a biological tissue-derived cell and a cell originated from the biological tissue-derived cell, or the like; can be particularly preferably an epithelial tissue-derived cell and a cell originated from the epithelial tissue cell, or the like, or a connective tissue-derived cell and a cell originated from the connective tissue-derived cell, or the like, or a muscle tissue-derived cell and a cell originated from the muscle tissue-derived cell, or the like, or a nerve tissue-derived cell and a cell originated from the nerve tissue-derived cell, or the like; can be even more preferably an animal-derived stem cell and a cell differentiated from the animal-derived stem cell, or the like; can be still more preferably an animal-derived pluripotent stem cell and a cell differentiated from the animal-derived pluripotent stem cell, or the like; can be yet more preferably a mammalian animal-derived pluripotent stem cell and a cell differentiated from the mammalian animal-derived pluripotent stem cell, or the like; and can be most preferably a human-derived pluripotent stem cell and a cell differentiated from the human-derived pluripotent stem cell, or the like.

In the present invention, a "stem cell" is a cell that can differentiate into another cell and has a self-renewal ability. Among "stem cells," cells, which have multilineage potential (pluripotency) capable of differentiating into all kinds of cells constituting the biological body and can continue to grow indefinitely while maintaining their pluripotency in in vitro culture under appropriate conditions, are particularly called "pluripotent stem cells." Specific examples of pluripotent stem cells include, but are not limited to, embryonic stem cells (ES cells), EG cells which are pluripotent stem cells derived from fetal primordial germ cells (Shamblott M. J. et al., Proc. Natl. Acad. Sci. USA. (1998) 95, p. 13726-13731), GS cells which are pluripotent stem cells derived from testicles (Conrad S., Nature (2008) 456, p. 344-349), induced pluripotent stem cells (IPS cells) which are induced pluripotent stem cells derived from somatic cells, and the like.

Pluripotent stem cells used in the present invention are particularly preferably ES cells or iPS cells. ES cells are pluripotent stem cells derived from early embryos. iPS cells are cultured cells to which pluripotency have been imparted after reprogramming somatic cells into an undifferentiated state by introducing a reprogramming factor into the somatic cells. As the reprogramming factor, it is possible to use, for example, OCT3/4 and KLF4 and SOX2 and c-Myc (Takahashi K, et al. Cell. 2007; 131: 861-72.), and it is possible to use, for example, OCT3/4 and SOX2 and LIN28 and Nanog (Yu J, et al. Science. 2007; 318: 1917-20.). A form of introducing these factors into cells is not particularly limited, and examples thereof include gene introduction using a plasmid, introduction of synthetic RNA, direct introduction as a protein, and the like. Furthermore, iPS cells, which are produced by a method using microRNA, RNA, low-molecular-weight compounds, the like, may be used. As pluripotent stem cells including ES cells and iPS cells, commercially available cells or cells for sale may be used, or newly produced cells may be used. As iPS cells, it is possible to use, for example, cell lines 253G1, 201B6, 201B7, 409B2, 454E2, HiPS-RIKEN-1A, HiPS-RIKEN-2A, HiPS-RIKEN-12A, Nips-B2, TkDN4-M, TkDA3-1, TkDA3-2, TkDA3-4, TkDA3-5, TkDA3-9, TkDA3-20, hiPSC38-2, MSC-iPSC1, BJ-iPSC1, and the like. As ES cells, it is possible to use, for example, cell lines KhES-1, KhES-2, KhES-3, KhES-4, KhES-5, SEES-1, SEES-2, SEES-3, SEES-4, SEES-5, SEES-6, SEES-7, HUES8, CyT49, HI, H9, HS-181, RPChiPS771-2, and the like. Newly produced clinical grade iPS cells or ES cells may be used. The origin of cells when producing iPS cells is not particularly limited, but for example, fibroblasts or lymphocytes can be used.

According to the present invention, a cell aggregate can be obtained. A cell aggregate, which is also called a spheroid, is a mass-like cell population formed by three-dimensionally aggregating a plurality of cells. A cell aggregate is generally substantially spherical. Cells constituting a cell aggregate are not particularly limited as long as they are composed of one or more kinds of the above-mentioned cells. For example, a cell aggregate composed of pluripotent stem cells such as human pluripotent stem cells or human embryonic stem cells contains cells which express a pluripotent stem cell marker and/or are positive for a pluripotent stem cell marker. Examples of pluripotent stem cell markers include Alkaline Phosphatase, NANOG, OCT4, SOX2, TRA-1-60, c-Myc, KLF4, LIN28, SSEA-4, SSEA-1, and the like.

A pluripotent stem cell marker in the present invention can be detected by any detection method in the technical field. Examples of methods for detecting an expression marker include, but are not limited to, flow cytometry. When a cell that emits stronger fluorescence as compared to a negative control (isotype control) is detected in flow cytometry in which a fluorescently labeled antibody is used, the cell is determined to be "positive" for the marker. A percentage of cells that are positive for a fluorescently labeled antibody analyzed by flow cytometry is sometimes referred to as a positive rate. Furthermore, as the fluorescently labeled antibody, any antibody known in the technical field can be used. Examples thereof include, but are not limited to, an antibody labeled with fluorescein isothiocyanate (FITC), phycoerythrin (PE), allophycocyanin (APC), or the like.

When cells constituting a cell aggregate or cell population are pluripotent stem cells, a proportion (percentage) of cells which express a pluripotent stem cell marker and/or are positive for a pluripotent stem cell marker can be, for example, 80% or more, 90% or more, 91% or more, 92% or more, 93% or more, 94% or more, 95% or more, 96% or more, 97% or more, 98% or more, or 99% or more. An upper limit of the proportion of the cells is not particularly limited. It can be 100% or less, and may be 100%. A cell aggregate or cell population, in which a proportion of cells which express a pluripotent stem cell marker and/or are positive for a pluripotent stem cell marker is within the above-mentioned range, is in a undifferentiated state at a high proportion and is more homogeneous cell population. The pluripotent stem cell marker is synonymous with an undifferentiation marker, and both terms can be used interchangeably.

A dimension of a cell aggregate produced by one or more embodiments of the present invention is not particularly limited, but in the case of observation with a microscope, an upper limit of the dimension of the widest portion in an observation image is, for example, 1000 µm or less, 900 µm or less, 800 µm or less, 700 µm or less, 600 µm or less, 500 µm or less, 400 µm or less, 300 µm or less, or 200 µm or less. A lower limit is, for example, 50 µm or more, 60 µm or more, 70 µm or more, 80 µm or more, 90 µm or more, or 100 µm or more. A cell aggregate having such a dimensional range is preferable as a cell growth environment because oxygen and nutrient components are easily supplied to cells inside.

In a group of cell aggregates produced by one or more embodiments of the present invention, for example, 10% or more, 20% or more, 30% or more, 40% or more, 50% or more, 60% or more, 70% or more, 80% or more, or 90% or more of the cell aggregates constituting the group based on a weight can have a dimension within the above-mentioned range. In a group of cell aggregates which contains 20% or more of cell aggregates having a dimension within the above-mentioned range, each of individual cell aggregates is preferable as a cell growth environment because oxygen and nutrient components are easily supplied to cells inside. In a cell population produced by one or more embodiments of the present invention, a proportion (survival rate) of living cells among cells constituting the population is preferably, for example, 50% or more, 60% or more, 70% or more, 80% or more, or 90% or more. A cell population in which a survival rate is within the above-mentioned range is in a preferable state for cell growth.

Somatic cells in the present invention can be obtained by inducing differentiation from pluripotent stem cells, and are not particularly limited as long as they are somatic cells that can be present in the biological body. Examples thereof include somatic stem cells (mesenchymal stem cells, neural stem cells, and the like, which are derived from bone marrow, adipose tissue, dental pulp, placenta, egg membrane, umbilical cord blood, amniotic membrane, chorion membrane, or the like), nerve cells, glial cells, oligodendrocytes, Schwann cells, cardiac muscle cells, cardiac muscle progenitor cells, liver cells, hepatic progenitor cells, α cells, β cells, fibroblasts, cartilage cells, corneal cells, vascular endothelial cells, vascular endothelial progenitor cells, pericytes, skeletal muscle cells, megakaryocytes, hematopoietic stem cells, airway epithelial cells, germ cells, dendritic cells, eosinophils, mast cells, T cells, erythropoietin-producing cells, intestinal epithelium, alveolar epithelial cells, kidney cells, pancreatic cells, hepatocytes, and the like. These cells may be in a gene-introduced form, or in a form in which a target gene on a genome, and the like are knocked down.

Cells used in the present invention may be cells derived from any animal. For example, cells may be derived from rodents such as mice, rats, and hamsters; primates such as humans, gorillas, and chimpanzees; and mammals such as domestic animals or pet animals such as dogs, cats, rabbits, cattle, horses, sheep, and goats, but cells derived from humans are particularly preferable.

According to a production method of the present invention, a cell population of any of three germ layers such as endoderm cells, ectoderm cells, and mesoderm cells, and somatic cells obtained from the cell population are produced from pluripotent stem cells. Examples of the three germ layers include endoderm cells, mesoderm cells, and ectoderm cells. Examples of somatic cells include the examples described above.

Endoderm cells in the present invention have an ability to differentiate into tissues of organs such as the digestive tract, lungs, thyroid, pancreas, and liver; secretory gland cells that open into the digestive tract; peritoneum; pleura; larynx; eustachian tube; trachea; bronchus; urinary tract (bladder, most parts of the urethra, a part of the ureter); and the like, and may be generally referred to as definitive endoderm (DE). Differentiation from pluripotent stem cells into endoderm cells can be confirmed by measuring an expression level of a gene specific to endoderm cells. Examples of genes specific to endoderm cells include SOX17, FOXA2, CXCR4, AFP, GATA4, EOMES, and the like. In the present specification, the term definitive endoderm may be used as another word for expressing the endoderm cells.

Mesoderm cells in the present invention are differentiated into body cavity and the mesothelium that lines the body cavity, muscles, skeleton, skin dermis, connective tissue, heart, blood vessels (including vascular endothelium), blood (including blood cells), lymph vessels, spleen, kidneys, ureter, gonads (testicles, uterus, gonadal epithelium), and the like. Examples of genes specific to mesoderm cells include MESP1, MESP2, FOXF1, BRACHYURY, HAND1, EVX1, IRX3, CDX2, TBX6, MIXL1, ISL1, SNAI2, FOXC1, PDGFRα, and the like.

Ectoderm cells in the present invention form epidermis of the skin and epithelium at the end portion of the urethra in men, hair, nails, skin glands (including mammary glands and sweat glands), sensory organs (salivary glands including oral cavity, pharynx, nose, epithelium at the end portion of rectum) crystalline lens, and the like. Some ectoderm cells invaginate into grooves during a development stage and form a neural tube to also become the origin of neurons and melanocytes of the central nervous system such as the brain and the spinal cord. Ectoderm cells also form the peripheral nervous system. Examples of genes specific to ectoderm cells include FGF5, OTX2, SOX1, PAX6, and the like.

According to the production method of the present invention, somatic cells can be produced by further inducing differentiation of three germ layers obtained by inducing differentiation of the pluripotent stem cells. The somatic cells obtained from endoderm cells will be described below.

Primitive gut tube (PGT) cells in the present invention form the foregut, midgut, and hindgut. The midgut is connected to the yolk sac, and the extraembryonic allantoic membrane branches from the hindgut. Furthermore, the foregut forms the pharynx of the respiratory system. There are organs, such as stomach and intestines, which are formed by differentiation of a gut tube as it is, and organs, such as liver, gallbladder, and pancreas (spleen (lymphatic organ)), which are formed in the form of budding from a gut tube. Differentiation from endoderm cells into primitive gut tube cells can be confirmed by measuring an expression level of a gene specific to primitive gut tube cells. Examples of genes specific to primitive gut tube cells include HNF-1β, HNF-4α, and the like.

Posterior foregut (PFG) cells in the present invention are cells differentiated from primitive gut tube cells, and can be confirmed by measuring an expression level of a gene specific to posterior foregut cells. Examples of genes specific to posterior foregut cells include PDX1, HNF6, and the like.

Pancreatic progenitor (PP) cells in the present invention are cells differentiated from posterior foregut cells and are cells capable of differentiating into exocrine cells and endocrine cells of the pancreas. Differentiation of posterior foregut cells into pancreatic progenitor cells can be confirmed by measuring an expression level of genes specific to pancreatic progenitor cells. Examples of genes specific to pancreatic progenitor cells include PDX1, NKX6.1, and the like.

Endocrine progenitor (EP) cells in the present invention are cells differentiated from pancreatic progenitor cells and are cells capable of differentiating into endocrine cells (α cells, β cells, δ cells, ε cells, PP cells, and the like) of the pancreas. Differentiation into pancreatic progenitor cells can be confirmed by measuring an expression level of genes specific to pancreatic progenitor cells. Examples of genes specific to pancreatic progenitor cells include PDX1, NKX6.1, NeuroG3, NeuroD1, and the like.

Pancreatic β cells in the present invention are cells differentiated from endocrine progenitor cells and are cells that secrete insulin. Differentiation of endocrine progenitor cells into pancreatic β cells can be confirmed by measuring an expression level of genes specific to pancreatic β cells. Examples of genes specific to pancreatic β cells include insulin, NKX6.1, MAFA, PDX1, and the like.

According to the production method of the present invention, by using an endoderm cell population into which pluripotent stem cells are induced to differentiate, it is possible to obtain somatic cells that can be used for treatment of the digestive system such as pancreas, liver, stomach, and intestines. Hereinafter, one of embodiments in treatment of each digestive system will be exemplified, but the present invention is not limited thereto.

In the intestinal system, when intestinal progenitor cells such as crypto cells are obtained, they can be used for treatment of ulcerative colitis, Crohn's disease, short gut syndrome, and the like by transplanting them with a catheter or the like.

In the pancreatic system, when pancreatic β cells (which may be expressed as insulin-producing cells as another word) are obtained, they can be used for treatment of diabetes by transplanting them with a catheter or the like or by transplanting them by encapsulating them in an immunoisolation device or the like.

In the liver system, for example, when albumin-producing cells are obtained, they can be used for treatment of external wounds accompanied by massive bleeding by transplanting them with a catheter or the like or by transplanting them by encapsulating them in an immune blocking device or the like.

Furthermore, it is also possible to obtain therapeutic tissues in the digestive system such as pancreas, liver, stomach, and intestines by culturing using a polymer support carrier or the like. For example, when liver tissue is induced and obtained, it can be used for treatment of liver cancer, cirrhosis, acute liver failure, and metabolic liver diseases such as hemochromatosis. When lung cell tissue is obtained, it can be used for treatment of lung respiratory organ diseases such as cystic fibrosis and asthma by transplanting it to a target lesion. In the renal system, when tissues containing mesangial cells, renal tubular epithelial cells, glomerular cells, and the like are obtained, they can be used for treatment of renal insufficiency and nephritis, treatment of dialysis, and the like by directly transplanting them. Albumin-producing cells, blood coagulation factor-producing cells, and cells producing metabolic enzymes such as α1-antitrypsin are produced by obtaining cells of the liver system which are capable of metabolizing substances. The produced metabolic enzymes are directly injected or are administered by infusion, and thereby they can be used for treatment of deficiency diseases of proteins thereof. For example, by obtaining cells of the pancreatic system, such as pancreatic β cells, which are capable of metabolizing substances, the cells can be used for treatment of type I diabetes by directly injecting insulin produced by the pancreatic β cells.

Furthermore, any cell population of three germ layers obtained by the production method of the present invention and somatic cells obtained from the cell population can also be used for drug efficacy/toxicity evaluation of test substances and elucidation of the mechanism of the action, or analysis of the biological phenomenon mechanism.

In the present invention, it is possible to use isolated cells obtained after culturing while adhering or suspending. The "isolated cells" referred to herein are cells obtained when cells, which are a plurality of cells adhered as a group, are detached and dispersed. Isolation is a process of detaching and dispersing into, single cells, cells in a state of adhering to a culture container, a culture carrier, or the like or a cell population in which cells are in a state of adhering to each other. The cell population to be isolated may be in a state of being suspended in a liquid medium. A method of isolation is not particularly limited, but it is possible to suitably use a detachment agent (cell detachment enzyme such as trypsin or collagenase), a chelating agent such as ethylenediaminetetraacetic acid (EDTA), a mixture of the detachment agent and the chelating agent, and the like. The detachment agent is not particularly limited, and examples thereof include trypsin, Accutase (registered trademark), TrypLE^{™} Express Enzyme (Life Technologies Corporation), TrypLE^{™} Select Enzyme (Life Technologies Corporation), DISPASE (registered trademark), collagenase, and the like. In the present invention, it is also possible to suitably use cells cryopreserved after isolation.

In the present invention, the phrase "culturing while suspending" means growing cells in a suspended state in a liquid medium, and the abbreviation term "suspension culture" can be used as another word for expression. Cells in suspension culture are present as an aggregated cell mass in a liquid medium. Although not particularly limited, in the present invention, the suspension culture is used as a method for mass culture of cells by three-dimensionally culturing cells. A culture method used in maintenance culture of pluripotent stem cells is not limited thereto, and culture may be performed while adhering or suspending.

The suspension culture may be static culture or may be culture under a condition in which a liquid medium flows, but is preferably the culture under a condition in which a liquid medium flows. As the culture under a condition in which a liquid medium flows, culture under a condition in which a liquid medium flows to promote cell aggregation is preferable. Examples of the culture under a condition in which a liquid medium flows to promote cell aggregation include culture under a condition in which a liquid medium flows so that cells gather at one point due to stress (centrifugal force, centripetal force) caused by flow such as gyrating flow and rocking flow, and culture under a condition in which a liquid medium flows by linear reciprocating motion, where the culture utilizing gyrating flow and/or rocking flow is particularly preferable.

In the present invention, a "gyratory culture method" (including a shaking culture method) refers to a method of culturing under a condition in which a liquid medium flows so that cells gather at one point due to stress (centrifugal force, centripetal force) caused by gyrating flow. Specifically, the method is performed by gyrating a culture container accommodating a liquid medium containing cells along approximately a horizontal plane in a closed orbit such as a circle, an ellipse, a flat circle, or a flat ellipse, or by gyrating a liquid medium in a culture container using a stirrer such as a stirring bar or a stirring blade while leaving the container to stand. The latter case can be achieved by, for example, using spinner flask-shaped culture containers to which a stirring blade is attached. Such culture containers are commercially available or produced by order, and they can also be used. In this case, regarding an amount of a liquid medium or culture solution, and the like, it is sufficient to use an amount recommended by a manufacturer of a culture container or an amount within a range that can be conceived of by those skilled in the art.

A gyrating speed in the gyratory culture method is not particularly limited, but an upper limit can be, for example, 200 rpm or less, 150 rpm or less, 120 rpm or less, 115 rpm or less, 110 rpm or less, 105 rpm or less, 100 rpm or less, 95 rpm or less, or 90 rpm or less. A lower limit can be, for example, 1 rpm or more, 10 rpm or more, 50 rpm or more, 60 rpm or more, 70 rpm or more, 80 rpm or more, or 90 rpm or more. A gyrating width during the gyratory culture is not particularly limited, but a lower limit can be, for example, 1 mm or more, 10 mm or more, 20 mm or more, or 25 mm or more. An upper limit of the gyrating width can be, for example, 200 mm or less, 100 mm or less, 50 mm or less, 30 mm or less, or 25 mm or less. A radius of rotation during the gyratory culture is also not particularly limited, but a gyrating width is set to be within the above-mentioned range. A lower limit of the radius of rotation is, for example, 5 mm or more, or 10 mm or more, and an upper limit thereof can be, for example, 100 mm or more, or 50 mm or more.

In the present invention, a "rocking culture method" refers to a method of culturing under a condition in which rocking flow is applied to a liquid medium by a linear reciprocating motion such as rocking stirring. Specifically, the method is performed by rocking a culture container accommodating a liquid medium containing cells in a plane substantially vertical to a horizontal plane. A rocking speed is not particularly limited, but for example, when one round trip is one time, it is sufficient for rocking to be performed 2 times or more, 4 times or more, 6 times or more, 8 times or more, or 10 times or more per minute as lower limits, and 15 times or less, 20 times or less, 25 times or less, or 50 times or less per minute as upper limits. When rocking, it is preferable to provide, to a culture container, a slight angle with respect to a vertical plane, that is, an angle of induction. A rocking angle is not particularly limited, but for example, a lower limit can be 0° or more, 1° or more, 2° or more, 4° or more, 6° or more, or 8° or more, whereas an upper limit can be 10° or less, 12° or less, 15° or less, 18° or less, or 20° or less. It is preferable to set conditions for the rocking culture within this range because then it is possible to produce cell aggregates having appropriate dimensions.

A seeding density of cells in a liquid medium in the suspension culture (cell density at the start of the suspension culture) can be appropriately adjusted, but a lower limit of the seeding density is, for example, 0.01 × 10⁵ cells/mL or more, 0.1 × 10⁵ cells/mL or more, or 1 × 10⁵ cells/mL or more. An upper limit of the seeding density is, for example, 10 × 10⁶ cells/mL or less, 20 × 10⁵ cells/mL or less, or 10 × 10⁵ cells/mL or less. When the seeding density is within this range, cell aggregates having appropriate sizes are easily formed. The seeding density may be, for example, 0.1 × 10⁵ cells/mL, 0.2 × 10⁵ cells/mL, 0.3 × 10⁵ cells/mL, 0.4 × 10⁵ cells/mL, 0.5 × 10⁵ cells/mL, 0.6 × 10⁵ cells/mL, 0.7 × 10⁵ cells/mL, 0.8 × 10⁵ cells/mL, 0.9 × 10⁵ cells/mL, 1 × 10⁵ cells/mL, 1.5 × 10⁵ cells/mL, 2 × 10⁵ cells/mL, 3 × 10⁵ cells/mL, 4 × 10⁵ cells/mL, 5 × 10⁵ cells/mL, 6 × 10⁵ cells/mL, 7 × 10⁵ cells/mL, 8 × 10⁵ cells/mL, 9 × 10⁵ cells/mL, or 10 × 10⁶ cells/mL.

Regarding the extent to which cells are grown in the suspension culture, or how to prepare the morphology and state of cells, it is sufficient to appropriately determine them according to the type and properties of cells to be cultured, the purpose of culture, the type of liquid medium, and culture conditions. Cells used for the suspension culture are preferably cells that have been cultured in a maintenance culture process in advance, collected in a collection process, and, if necessary, dissociated into single cells. After the suspension culture, a culture solution is discarded by a conventional method, and the cells are collected. At this time, it is preferable that the cells be collected as single cells by detaching or dispersion treatment. It is sufficient for the collected cells to be provided to the next process as they are or, if necessary, after washing them with a buffer, physiological saline, or a liquid medium.

A liquid medium used in the present invention is a liquid substance adjusted for culturing cells. As a general rule, the liquid medium contains the required minimum amount or more of components essential for cell growth and/or maintenance. For example, the liquid medium can be prepared by using any medium for animal cell culture as a basal medium and appropriately adding other components such as culture additives if necessary. The liquid medium used in the present invention is preferably a liquid medium suitable for the suspension culture of cells. A solid medium, which is partially or wholly gelled, stays in a liquid sending tube when being sent, and induces morphological changes and changes in concentration of main components as the temperature of the medium changes. Accordingly, the medium used in the present invention is a liquid substance. In the present specification, the term medium may be used as simple reference of the liquid medium.

A viscosity of the liquid medium in the present invention is not particularly limited as long as the viscosity is within a range that enables the liquid medium to be recognized as a liquid by those skilled in the art. Specifically, when the temperature of the liquid medium is 25°C, a viscosity is preferably 100 mPa·s or less, 90 mPa·s or less, 80 mPa·s or less, 70 mPa·s or less, 60 mPa·s or less, 50 mPa·s or less, 40 mPa·s or less, or 30 mPa·s or less, and is preferably more than 0 mPa·s, 1 mPa·s or more, 2 mPa·s or more, 3 mPa·s or more, 4 mPa·s or more, or 5 mPa·s or more.

The viscosity of the liquid medium is not particularly limited as long as it is a viscometer capable of measuring the viscosity of the liquid medium. For example, measurement can be performed by a B-type viscometer (TOKIMEC, INC.). Specifically, a measurement sample is put into a glass container having an inner diameter of 60 mm; measurement is performed three times under conditions of a liquid temperature of 25°C, a rotor No. 2, a rotation speed of 60 rotations/minute, and a retention time of 30 seconds; and an average value of the measurements can be used as a measured value (viscosity).

In the present invention, the phrase "not added" means that factors such as proteins, peptides, and compounds, which are identified to be not added in a culture or a conditioned medium, are not extrinsically added. When factors such as proteins, peptides, and compounds, which are identified to be not added in a culture or a conditioned medium, are brought in by continuous operation of the culture, an amount thereof is adjusted to less than 1% (volume/volume), less than 0.5% (volume/volume), less than 0.1% (volume/volume), less than 0.05% (volume/volume), less than 0.01% (volume/volume), or less than 0.001% (volume/volume).

As a basal medium, it is possible to use media such as an BME medium, an BGJb medium, a CMRL1066 medium, a Glasgow MEM medium, an Improved MEM Zinc Option medium, an Iscove's Modified Dulbecco's Medium (IMDM medium), a Medium 199 medium, an Eagle MEM medium, an αMEM medium, a Dulbecco's Modified Eagle's Medium (DMEM medium), a Ham's F10 medium, a Ham's F12 medium, an RPMI 1640 medium, a Fischer's medium, and a mixture of these media (for example, a Dulbecco's Modified Eagle's Medium/Nutrient Mixture F-12 Ham (DMEM/F12 medium)), but media are not particularly limited. As the DMEM/F12 medium, in particular, it is possible to use a medium obtained by mixing a DMEM medium and a Ham's F12 medium in, for example, a weight ratio of 60/40 or more and 40/60 or less, a weight ratio of 55/45 or more and 45/55 or less, or an equal amount (weight ratio of 50/50).

In the present invention, a "culture additive" is a substance which is added to the medium for the purpose of culturing except serum. Specific examples of culture additives are not particularly limited. Examples thereof include L-ascorbic acid, insulin, transferrin, selenium, sodium hydrogen carbonate, growth factors, fatty acids or lipids, amino acids (for example, non-essential amino acids), vitamins, cytokines, antioxidant agents, 2-mercaptoethanol, pyruvic acid, buffering agents, inorganic salts, antibiotics, and the like. Insulin, transferrin, and cytokines may be of natural origin separated from tissues or sera, and the like of animals (preferably humans, mice, rats, cattle, horses, goats, and the like), or may be a recombinant protein produced by genetic engineering. Furthermore, regarding growth factors, although there is no limitation, it is possible to use, for example, Basic fibroblast growth factor-2 (FGF2), Transforming growth factor-β1 (TGF-β1), Activin A, IGF-1, MCP-1, IL-6, PAI, PEDF, IGFBP-2, LIF, and IGFBP-7. Regarding antibiotics, although there is no limitation, it is possible to use, for example, penicillins, streptomycin, amphotericin B, and the like.

The liquid medium used in the present invention preferably contains at least one selected from L-ascorbic acid, insulin, transferrin, selenium, and sodium hydrogen carbonate, and more preferably contains all of them. In addition, L-ascorbic acid, insulin, transferrin, selenium, and sodium hydrogen carbonate can be added to the medium in the form of solutions, derivatives, salts, mixed reagents, and the like. For example, L-ascorbic acid may be added to the medium in the form of a derivative such as magnesium ascorbic acid-2-phosphate. Selenium may be added to the medium in the form of selenite (such as sodium selenite). Insulin and transferrin may be of natural origin separated from tissues or sera, and the like of animals (preferably humans, mice, rats, cattle, horses, goats, and the like), or may be a recombinant protein produced by genetic engineering. Insulin, transferrin, and selenium may be added to the medium in the form of a reagent ITS (insulin-transferrin-selenium). ITS is a cell growth-promoting additive containing insulin, transferrin, and sodium selenite. It is possible to use commercially available media containing at least one selected from L-ascorbic acid, insulin, transferrin, selenium, and sodium hydrogen carbonate. As commercially available media into which insulin and transferrin are added, it is possible to use CHO-S-SFM II (Life Technologies Corporation), Hybridoma-SFM (Life Technologies Corporation), eRDF Dry Powdered Media (Life Technologies Corporation), UltraCULTURETM (BioWhittaker, Inc.), UltraDOMA^{™} (BioWhittaker, Inc.), UltraCHO^{™} (BioWhittaker, Inc.), UltraMDCK^{™} (BioWhittaker, Inc.), and the like. It is also possible to suitably use STEMPRO (registered trademark) hESC SFM (Life Technologies Corporation), mTeSR1 (VERITAS Corporation), TeSR2 (VERITAS Corporation), and the like. In addition, it is also possible to suitably use media used for culturing human iPS cells and human ES cells.

In the present invention, the "culture container" is not particularly limited as long as it has a shape, size, structure, and configuration in which pluripotent stem cells can be cultured in a suspended state, but the culture container preferably has a shape, volume, structure, and configuration which reduce adhesiveness of cells to an inner surface of the container. In the present specification, the term culture tank may be used as another word for expressing the culture container.

The shape of the culture container is not particularly limited. For example, culture containers, which have a shape such as a tank shape, a flow reactor shape, a dish shape, a flask shape, a well shape, and a bag shape, are exemplified. A volume of the culture container used in the present invention can be appropriately selected and is not particularly limited, but an area, when a bottom surface of a portion accommodating the medium is viewed in a plan view, is preferably, for example, 25 cm² or larger, 50 cm² or larger, 100 cm² or larger, 200 cm² or larger, 300 cm² or larger, 400 cm² or larger, 500 cm² or larger, 600 cm² or larger, 700 cm² or larger, 800 cm² or larger, 900 cm² or larger, 1000 cm² or larger, 2000 cm² or larger, 3000 cm² or larger, 4000 cm² or larger, 5000 cm² or larger, 6000 cm² or larger, 7000 cm² or larger, 8000 cm² or larger, 9000 cm² or larger, or 10000 cm² or larger, or is preferably, for example, 1000000 cm² or smaller, 500000 cm² or smaller, 100000 cm² or smaller, 90000 cm² or smaller, 80000 cm² or smaller, 70000 cm² or smaller, 60000 cm² or smaller, 50000 cm² or smaller, 40000 cm² or smaller, 30000 cm² or smaller, 20000 cm² or smaller, 10000 cm² or smaller, 9000 cm² or smaller, 8000 cm² or smaller, 7000 cm² or smaller, 6000 cm² or smaller, 5000 cm² or smaller, 4000 cm² or smaller, 3000 cm² or smaller, 2000 cm² or smaller, or 1000 cm² or smaller.

A volume of the liquid medium (L/culture container) used in the present invention is not particularly limited as long as it is a volume that enables suspension culture of pluripotent stem cells and formation of cell aggregates. For example, a volume is preferably 0.1 L/culture container or more, 0.5 L/culture container or more, 1 L/culture container or more, 2 L/culture container or more, 3 L/culture container or more, 4 L/culture container or more, 5 L/culture container or more, 6 L/culture container or more, 7 L/culture container or more, 8 L/culture container or more, 9 L/culture container or more, 10 L/culture container or more, 20 L/culture container or more, 30 L/culture container or more, 40 L/culture container or more, 50 L/culture container or more, 60 L/culture container or more, 70 L/culture container or more, 80 L/culture container or more, 90 L/culture container or more, or 100 L/culture container or more, and is preferably 100000 L/culture container or less, 50000 L/culture container or less, 10000 L/culture container or less, 9000 L/culture container or less, 8000 L/culture container or less, 7000 L/culture container or less, 6000 L/culture container or less, 5000 L/culture container or less, 4000 L/culture container or less, 3000 L/culture container or less, 2000 L/culture container or less, 1000 L/culture Container or less, 900 L/culture container or less, 800 L/culture container or less, 700 L/culture container or less, 600 L/culture container or less, 500 L/culture container or less, 400 L/culture container or less, 300 L/culture container or less, 200 L/culture container or less, 150 L/culture container or less, 100 L/culture container or less, 90 L/culture container or less, 80 L/culture container or less, 70 L/culture container or less, 60 L/culture container or less, or 50 L/culture container or less.

In the present invention, a "maintenance culture process" is a process of culturing, to grow cells in a state where an undifferentiated state is maintained, a cell population before suspension culture or a cell aggregate obtained after suspension culture or after a collection process thereafter. The maintenance culture may be adhesion culture in which cells are cultured while being adhered to a culture substrate such as a container and a carrier, or may be suspension culture in which cells are cultured while being suspended in a liquid medium.

In the maintenance culture process, it is sufficient for target cells to be cultured by an animal cell culture method known in the field. Any of the adhesion culture or the suspension culture may be used.

Specific embodiments of a liquid medium, cells, a seeding density of cells, a culture container, the shape of the culture container, and culture conditions, which are used in the maintenance culture process are as described above.

A flow state of a liquid medium in the maintenance culture process is not particularly limited, but static culture may be used or flow culture may be used.

The "static culture" refers to culture performed in a culture container with a liquid medium being in a static state. In the adhesion culture, this static culture is generally adopted.

The "flow culture" refers to culturing under a condition in which a liquid medium is caused to flow. Specific embodiments of the flow culture are as described above.

Regarding the extent to which cells are grown in the maintenance culture process, or how to prepare the morphology and state of cells, it is sufficient to appropriately determine them according to the type and properties of cells to be cultured, the purpose of culture, the type of liquid medium, and culture conditions.

In the maintenance culture process, it is preferable to exchange a liquid medium at an appropriate frequency. A frequency of exchanging a liquid medium varies depending on the type of cell, but a liquid medium exchange operation can be included at a frequency of, for example, one time or more every 5 days, one time or more every 4 days, one time or more every 3 days, one time or more every 2 days, or one time or more every 1 day. Liquid medium exchange at these frequencies is particularly suitable when culturing cell aggregates of stem cells. A method of exchanging a liquid medium is not particularly limited. Preferably, a total amount of a cell culture composition containing cell aggregates is collected in a centrifuge tube, and is subjected to centrifugal separation or left to stand in a static state for 5 minutes; the supernatant is removed while leaving the precipitated cell aggregates; a fresh liquid medium is added thereafter to gently disperse the cell aggregates; thereafter, the dispersion medium of the cell aggregates is again returned to a culture container such as a plate; and thereby culturing of the cell aggregates can be continued. A culture period in the maintenance culture process of the present aspect is not particularly limited, but is preferably 3 days or longer and 7 days or shorter.

After the maintenance culture process, a culture solution is discarded by a conventional method, and cells are collected. At this time, it is preferable that the cells be collected as single cells by detaching or dispersion treatment. It is sufficient for the collected cells to be provided to the next process as they are or, if necessary, after washing them with a buffer (including a PBS buffer), physiological saline, or a liquid medium (where a liquid medium to be used in the next process is preferably a basal medium).

The "collection process" is a process of collecting cultured cells from a culture solution after the suspension culture and/or the maintenance culture process, and is a selection process in the method of the present invention.

In the present invention, "collection (of cells)" means to acquire cells by separating a culture solution and cells. It is sufficient for a cell collection method to follow a conventional method used in a cell culture method in the field, and there is no particular limitation. The cell culture method can be generally roughly divided into a suspension culture method and an adhesion culture method. Hereinafter, the cell collection method after each of the culture methods will be described.

### (Collection method after suspension culture method)

When culture is performed by the suspension culture method, cells are present in a suspended state in a culture solution. Accordingly, cell collection can be achieved by removing a liquid component of the supernatant, from a static state or by centrifugal separation. Furthermore, as the cell collection method, it is possible to select a filter, a hollow fiber separation membrane, or the like. When removing a liquid component from a static state, it is sufficient to leave a container containing a culture solution in a static state for about 5 minutes to remove the supernatant while leaving the precipitated cells and cell aggregates. Furthermore, it is sufficient for centrifugal separation to be performed at a rotation speed and a treatment time at which cells are not damaged due to the centrifugal force. For example, a lower limit of the rotation speed is not particularly limited as long as cells can be precipitated, but the lower limit may be, for example, 500 rpm or more, 800 rpm or more, or 1000 rpm or more. Meanwhile, it is sufficient for an upper limit to be a speed at which cells are not damaged or are unlikely to be damaged due to centrifugal force. It is sufficient for the upper limit to be, for example, 1400 rpm or less, 1500 rpm or less, or 1600 rpm or less. Furthermore, a lower limit of the treatment time is not particularly limited as long as it is a time in which cells can be precipitated at the above-mentioned rotation speed. It is sufficient of the lower limit to be, for example, 30 seconds, 1 minute, 3 minutes, or 5 minutes. Furthermore, it is sufficient for an upper limit to be a time during which cells are not damaged or are unlikely to be damaged by the rotation. It is sufficient for the upper limit to be, for example, 30 seconds, 6 minutes, 8 minutes, or 10 minutes. The collected cells can be washed if necessary. A washing method is not limited. For example, it is sufficient to carry out washing in the same manner as a washing method described in "treatment after the process" in the above-described maintenance culture process. As a washing liquid, it is sufficient to use a buffer (including a PBS buffer), physiological saline, or a liquid medium (preferably a basal medium).

### (Collection method after adhesion culture method)

When culture is performed by the adhesion culture method, many cells are present in a state of being adhered to an external matrix such as a culture container or a culture carrier after culturing. Therefore, in order to remove a culture solution from a culture container, it is sufficient to gently tilt the container after culturing to flush out a liquid component. Because cells adhered to the external matrix remain in the culture container, the culture solution and the cells can be easily separated.

Thereafter, the cell surface adhered to the external matrix can be washed if necessary. As a washing liquid, it is sufficient to use a buffer (including a PBS buffer), physiological saline, or a liquid medium (preferably a basal medium). However, a washing liquid is not limited thereto. It is sufficient to remove a washing liquid after washing by the same operation as for the culture solution. This washing step may be repeated multiple times.

Subsequently, a cell population adhered to the external matrix is detached from the external matrix. It is sufficient to perform a detachment method by a method known in the field. In general, scraping, a detachment agent containing a proteolytic enzyme as an active ingredient, a chelating agent such as EDTA, a mixture of the detachment agent and the chelating agent, and the like are used.

Scraping is a method of stripping off cells adhered to an external matrix by mechanical means using a scraper or the like. However, since cells are easily damaged by a mechanical operation, when collected cells are provided to further culture, a detachment method is preferable, in which adhesion to an external matrix is resolved by chemically disrupting or decomposing a scaffolding portion of cells fixed to the external matrix.

In the detachment method, a detachment agent and/or a chelating agent is used. The detachment agent is not limited, but it is possible to use, for example, trypsin, collagenase, pronase, hyaluronidase, and elastase; and in addition, Accutase (registered trademark), TrypLE^{™} Express Enzyme (Life Technologies Corporation), TrypLE^{™} Select Enzyme (Life Technologies Corporation), and DISPASE (registered trademark), which are commercially available; and the like. It is sufficient for a concentration and a treatment time of each detachment agent to be used within a range of a conventional method used for detachment or dispersion of cells. For example, in the case of trypsin, a lower limit of a concentration in a solution is not particularly limited as long as it is a concentration at which cells can be detached. It is sufficient for the lower limit to be, for example, 0.01% or more, 0.02% or more, 0.03% or more, 0.04% or more, 0.05% or more, 0.08% or more, or 0.10% or more.

Meanwhile, an upper limit of a concentration in a solution is not particularly limited as long as it is a concentration at which there is no influence, such as lysis of cells themselves, on cells due to the action of trypsin. It is sufficient for the upper limit to be, for example, 0.15% or less, 0.20% or less, 0.25% or less, or 0.30% or less. Furthermore, although a treatment time depends on the concentration of trypsin, a lower limit thereof is not particularly limited as long as it is a time in which cells are sufficiently detached from an external matrix by the action of trypsin. It is sufficient for the lower limit to be, for example, 1 minute or longer, 2 minutes or longer, 3 minutes or longer, 4 minutes, or 5 minutes or longer. Meanwhile, an upper limit of the treatment time is not particularly limited as long as it is a time during which there is no influence, such as lysis of cells themselves, on cells due to the action of trypsin. It is sufficient for the upper limit to be, for example, 8 minutes or shorter, 10 minutes or shorter, 12 minutes or shorter, 15 minutes or shorter, 18 minutes or shorter, or 20 minutes or shorter. In a case of other detachment agents or chelating agents, it is sufficient for the detachment method to be performed in a similar manner. When a commercially available detachment agent is used, the detachment method can be carried out at a concentration and a treatment time described in the attached protocol. Cells collected after the present collection process can be dissociated into single cells if necessary.

In the present invention, "dissociation into single cells" means that a single free cell state is caused by dispersing a cell assembly, such as a monolayer cell fragment and a cell aggregate, in which a plurality of cells are adhered to each other or aggregated to each other.

For the dissociation into single cells, it is sufficient to increase a concentration of a detachment agent and/or a chelating agent used in the above-described detachment method, and/or lengthen a treatment time with the detachment agent and/or the chelating agent. For example, in the case of trypsin, a lower limit of a concentration in a solution is not particularly limited as long as it is a concentration at which a cell assembly can be dispersed. It is sufficient for the lower limit to be, for example, 0.15% or more, 0.18% or more, 0.20% or more, or 0.24% or more. Meanwhile, an upper limit of a concentration in a solution is not particularly limited as long as it is a concentration at which there is no influence, such as lysis of cells themselves, on cells. It is sufficient for the upper limit to be 0.25% or less, 0.28% or less, or 0.30% or less. Furthermore, although a treatment time depends on the concentration of trypsin, a lower limit thereof is not particularly limited as long as it is a time in which a cell assembly is sufficiently dispersed by the action of trypsin. It is sufficient for the lower limit to be, for example, 5 minutes or longer, 8 minutes or longer, 10 minutes or longer, 12 minutes or longer, or 15 minutes or longer. Meanwhile, an upper limit of the treatment time is not particularly limited as long as it is a time during which there is no influence, such as lysis of cells themselves, on cells due to the action of trypsin. It is sufficient for the upper limit to be, for example, 18 minutes or shorter, 20 minutes or shorter, 22 minutes or shorter, 25 minutes or shorter, 28 minutes or shorter, or 30 minutes or shorter.

When a commercially available detachment agent is used, it is sufficient for a detachment agent to be used at a concentration at which cells can be dispersed to be dissociated into a single state within the description in the attached protocol. The dissociation into single cells can be promoted by lightly performing physical treatment after the treatment with the detachment agent and/or the chelating agent. This physical treatment is not limited, and examples thereof include a method of pipetting cells together with a solution multiple times. Furthermore, cells may be passed through a strainer or mesh if necessary.

The cells dissociated into single cells can be collected by removing the supernatant containing the detachment agent by leaving the cells to stand or subjecting the cells to centrifugal separation. The collected cells may be washed if necessary. It is sufficient for conditions for centrifugal separation and a washing method to be as described above.

Specific embodiments of the present invention will be described below, but the present invention is not limited thereto. Furthermore, explanations described above will be omitted.

The production method of the present invention is a production method including the following (a) and (b):
(a) a process of filling a culture container with a liquid medium and thereafter raising, in the culture container, a temperature of the liquid medium to a temperature at which pluripotent stem cells grow; and
(b)a process of seeding pluripotent stem cells in the liquid medium in the culture container to culture the pluripotent stem cells while suspending the pluripotent stem cells.

The process (a), which is the process of filling a culture container with a liquid medium and thereafter raising, in the culture container, a temperature of the liquid medium to a temperature at which pluripotent stem cells can proliferate, corresponds to a stage prior to a process of suspension culture of pluripotent stem cells. It is preferable to continuously perform the present process and the process (b).

In the process (a), when filling a culture container with an unheated liquid medium (a liquid medium maintained at a temperature at which it has been stored), a worker may directly fill the culture container with the liquid medium; and when a culture container, and a container in which a liquid medium is stored are connected by a liquid sending tube such as a pipe, the culture container may be filled by sending the liquid from the container to the container using air pressure or the like in a sterile environment. A temperature of the liquid medium when filling the culture container therewith is not particularly limited as long as it is a temperature at which components contained in the liquid medium can be stably present for at least 6 months. The temperature is preferably, for example, -80°C or higher, -70°C or higher, -60°C or higher, -50°C or higher, -40°C or higher, -30°C or higher, -20°C or higher, -10 or higher, -5°C or higher, 0°C or higher, or 4°C or higher, and is preferably, for example, 18°C or lower, 15°C or lower, 10°C or lower, 9°C or lower, 8°C or lower, 7°C or lower, 6°C or lower, or 5°C or lower. Furthermore, as another embodiment, a temperature within the culture container may be lowered to -20°C after filling it with a liquid medium at, for example, 4°C, and thereby setting a temperature of the liquid medium to -20°C in order to make the filling operation efficient and stabilize a composition of the liquid medium. That is, it is preferable to appropriately adjust temperatures of a liquid medium at the time of filling and a liquid medium before heating within a temperature range in which components contained in the liquid medium described above are stabilized.

Next, in the process (a), a temperature of the liquid medium is raised in the culture container to a temperature at which pluripotent stem cells can proliferate. A temperature of the liquid medium in the culture container before raising the temperature may be the same as a temperature of the liquid medium at the time of filling, or may be changed within a temperature range in which components contained in the liquid medium are stabilized. Specifically, the temperature is preferably -80°C or higher, -70°C or higher, -60°C or higher, -50°C or higher, -40°C or higher, -30°C or higher, -20°C or higher, -10 or higher, -5°C or higher, 0°C or higher, or 4°C or higher, and is preferably 18°C or lower, 15°C or lower, 10°C or lower, 9°C or lower, 8°C or lower, 7°C or lower, 6°C or lower, or 5°C or lower. In particular, when a temperature of the liquid medium in the culture container before raising the temperature is -20°C or higher and 10°C or lower, this is preferable because then growth of pluripotent stem cells is significantly improved.

Raising (a range within which a temperature of the liquid medium is raised) of the temperature of the liquid medium in the process (a) can be appropriately selected and is not particularly limited. For example, the temperature is raised by 15°C, is raised by 16°C, is raised by 17°C, or is raised by 18°C. It is preferable that the temperature be raised by 19°C, raised by 20°C, raised by 21°C, raised by 22°C, raised by 23°C, raised by 24°C, raised by 25°C, raised by 26°C, raised by 27°C, raised by 28°C, raised by 29°C, raised by 30°C, raised by 31°C, raised by 32°C, raised by 33°C, raised by 34°C, raised by 35°C, raised by 36°C, raised by 37°C, raised by 38°C, raised by 39°C, raised by 40°C, raised by 41°C, raised by 42°C, raised by 43°C, raised by 44°C, raised by 45°C, raised by 46°C, raised by 47°C, raised by 48°C, raised by 49°C, raised by 50°C, raised by 51°C, raised by 52°C, raised by 53°C, raised by 54°C, raised by 55°C, raised by 56°C, raised by 57°C, raised by 58°C, raised by 59°C, or raised by 60°C. In particular, as a range within which a temperature of the liquid medium is raised, it is preferable to raise the liquid medium within a range of 20°C or higher and 57°C or lower, it is more preferable to raise the liquid medium within a range of 22°C or higher and 57°C or lower, and it is particularly preferable to raise the liquid medium within a range of higher than 37°C and 57°C or lower.

A temperature at which pluripotent stem cells can proliferate in the process (a) is not particularly limited as long it is a temperature at which pluripotent stem cells can survive and grow. The temperature is preferably, for example, 30°C or higher, 31°C or higher, 32°C or higher, 33°C or higher, 34°C or higher, or 35°C or higher, and is preferably, for example, 42°C or lower, 41°C or lower, 40°C or lower, 39°C or lower, 38°C or lower, or 37°C or lower.

In the process (a), it is preferable to raise the temperature of the liquid medium while stirring the liquid medium in the culture container. Examples of embodiments for stirring the liquid medium in the culture container include stirring under a condition in which a liquid medium flows due to stress (centrifugal force, centripetal force) caused by flow such as gyrating flow and rocking flow, and stirring under a condition in which a liquid medium flows by linear reciprocating motion, where the stirring utilizing gyrating flow and/or rocking flow is preferable. More specifically, the temperature of the liquid medium in the culture container can be efficiently raised by gyrating the liquid medium in the container using a stirrer such as a stirring blade.

It is preferable that a growth factor be not added in the process (a). It has been found by the present invention that a temperature change in the liquid medium in the present process induces destabilization of growth factors. Examples of growth factors include FGF2, TGF-β1, Activin A, IGF-1, MCP-1, IL-6, PAI, PEDF, IGFBP-2, LIF, and IGFBP-7, where FGF2 and/or TGF-β1 is particularly preferable.

The process (b), which is the process of seeding pluripotent stem cells in the liquid medium in the culture container to culture the pluripotent stem cells while suspending the pluripotent stem cells, corresponds to a stage subsequent to the process of raising the temperature of the liquid medium in the culture container. It is preferable to continuously perform the process (a) and the present process.

As a method for culturing pluripotent stem cells while suspending them in the process (b), it is possible to use the above-described suspension culture method, or it is possible to use a method disclosed in Japanese Patent No. 6238265.

It is preferable to add a growth factor in the process (b). It has been found by the present invention that pluripotent stem cells grow efficiently by adding a growth factor in the present process. Examples of growth factors include FGF2, TGF-β1, Activin A, IGF-1, MCP-1, IL-6, PAI, PEDF, IGFBP-2, LIF, and IGFBP-7, where FGF2 and/or TGF-β1 is particularly preferable. A concentration of a growth factor added is not particularly limited as long as it is within a concentration range in which pluripotent stem cells can proliferate. The concentration is preferably 1 µg/L or more, 3 µg/L or more, 5 µg/L or more, 7 µg/L or more, or 10 µg/L or more, and is preferably 500 µg/L or less, 400 µg/L or less, 300 µg/L or less, 200 µg/L or less, or 100 µg/L or less.

Furthermore, in the production method of the present invention, somatic cells can be obtained by performing the following process (c) using the pluripotent stem cells obtained after the above-described process (a) and process (b).

A production method for somatic cells of the present invention is a production method including the following (a) to (c):
(a) a process of filling a culture container with a liquid medium and thereafter raising, the temperature of the liquid medium in the culture container to the temperature at which pluripotent stem cells can proliferate;
(b) a process of seeding pluripotent stem cells in the liquid medium in the culture container and culturing the pluripotent stem cells in suspension; and
(c) a process of culturing the pluripotent stem cells obtained in the process (b) in the presence of a differentiation-inducing factor to induce differentiation.

The process (c), which is the process of culturing the pluripotent stem cells obtained in the process (b) in the presence of a differentiation-inducing factor to induce differentiation, corresponds to a stage subsequent to the process of performing suspension culture of pluripotent stem cells. It is preferable to continuously perform the process (b) and the present process.

Examples of the differentiation-inducing factor in the process (c) include a substance that acts on TGFβ signaling, a substance that acts on Wnt signaling, a substance that acts on Hedgehog signaling, a substance that acts on BMP signaling, and a substance that acts on Nodal/Activin signaling. Specifically, it is possible to use differentiation-inducing factors disclosed in PCT International Publication No. WO2016/063986, PCT International Publication No. WO2012/020845, and PCT International Publication No. WO2016/060260.

A cell population containing the somatic cells according to the present invention can be used as a pharmaceutical product composition. That is, according to the present invention, there is provided a pharmaceutical composition containing: the somatic cells according to the present invention and a cell population containing the somatic cells, and a pharmaceutically acceptable medium. According to the present invention, there is further provided a pharmaceutical composition containing: the somatic cells according to the present invention and a cell population containing the somatic cells, and other cells that can be administered.

The pharmaceutical composition of the present invention can be used as a cell therapeutic agent, for example, a therapeutic agent for intractable diseases. According to the present invention, there is provided a method for transplanting cells into a patient or subject, the method including a process of administering, to the patient or subject, a therapeutically effective amount of a cell population containing the somatic cells according to the present invention; and a method for treating a disease of a patient or subject.

According to the present invention, use of a cell population containing the somatic cells according to the present invention is provided for production of the pharmaceutical composition.

According to the present invention, use of a cell population containing the somatic cells according to the present invention is provided for production of a cell therapeutic agent.

The pharmaceutical composition of the present invention may be a pharmaceutical composition in which a cell population containing the somatic cells is diluted with a pharmaceutically acceptable medium. The above-mentioned pharmaceutically acceptable medium is not particularly limited as long as it is a solution that can be administered to a patient or subject. The pharmaceutically acceptable medium may be an infusion formulation. Examples thereof include, but are not limited to, water for injection, physiological saline, 5% dextrose in water solution, a Ringer's solution, a Ringer's lactate solution, a Ringer's acetate solution, a Ringer's bicarbonate solution, an amino acid solution, a starting fluid (fluid No. 1), a dehydration replenishment fluid (fluid No. 2), a maintenance infusion (fluid No. 3), a postoperative collection fluid (fluid No. 4), Plasma-Lyte A (registered trademark), and the like.

In the present invention, the "patient or subject" is typically a human, but may be another animal. Examples of other animals include, but are not limited to, mammals such as dogs, cats, cattle, horses, pigs, goats, sheep, monkeys (crab-eating macaque, rhesus macaque, Callithrix jacchus, Japanese macaque), ferrets, rabbits, and rodents (mice, rats, gerbils, guinea pigs, hamsters); and birds such as chickens and quails.

The "treatment" in the present invention means that at least one of the following examples is significantly ameliorated: life prognosis, functional prognosis, survival rate, body weight loss, anemia, diarrhea, melena, abdominal pain, fever onset, loss of appetite, malnutrition, emesis, fatigue, rash, inflammation, ulcer, erosion, fistula, stenosis, blockage of the intestines, internal bleeding, rectal bleeding, convulsion, aching pain, deterioration in liver function, or blood test items of a patient or subject. However, examples are not limited thereto.

The pharmaceutical composition of the invention may contain any ingredient used in the treatment of a patient or subject. Examples of the above-mentioned ingredients include, but are not limited to, salts (for example, physiological saline, Ringer's solution, BICANATE injection), polysaccharides (for example, hydroxyethyl starch (HES), dextran, and the like), proteins (for example, albumin), dimethyl sulfoxide (DMSO), amino acids, medium components (for example, components contained in RPMI 1640 medium, and the like), and the like.

The pharmaceutical composition of the present invention may contain various additives such as emulsifiers, dispersants, buffering agents, preservatives, wetting agents, antioxidant agents, chelating agents, thickeners, gelling agents, and pH adjusters to increase preservation stability, isotonicity, absorbency, and/or viscosity. Examples of thickeners include, but are not limited to, HES, dextran, methyl cellulose, xanthan gum, carboxymethyl cellulose, hydroxypropyl cellulose, and the like. A concentration of a thickener depends on a thickener selected, but can be arbitrarily set within a range of concentrations which are safe in the case of administration to a patient or subject and at which a desired viscosity is achieved.

The pharmaceutical composition of the present invention may contain one or a plurality of other pharmaceuticals in addition to somatic cells. Examples of the above-mentioned other pharmaceuticals include, but are not limited to, antibiotics, albumin formulations, vitamin formulations, anti-inflammatory agents, and the like. Examples of the above-mentioned anti-inflammatory agents include, but are not limited to, 5-aminosalicylic acid formulations, steroid formulations, immunosuppressive agents, biological formulations, and the like. Examples of the above-mentioned 5-aminosalicylic acid formulations include, but are not limited to, salazosulfapyridine, mesalazine, and the like. Examples of the above-mentioned steroid formulations include, but are not limited to, cortisone, prednisolone, methylprednisolone, and the like. Examples of the above-mentioned immunosuppressive agents include tacrolimus, cyclosporine, methotrexate, azathioprine, 6-mercaptopurine, and the like. Examples of the above-mentioned biological formulations include, but are not limited to, infliximab, adalimumab, ustekinumab, secukinumab, ixekizumab, brodalumab, tocilizumab, vedolizumab, filgotinib, golimumab, certolizumab pegol, abatacept, etanercept, and the like. Furthermore, the above-mentioned other pharmaceuticals may be cells of other types.

A pH of the pharmaceutical composition of the present invention can be near neutral pH, for example, pH 5.5 or more, pH 6.0 or more, pH 6.5 or more, or pH 7.0 or more, and can be pH 10.5 or less, pH 9.5 or less, pH 8.5 or less, or pH 8.0 or less, but the pH is not limited thereto.

A cell concentration of the pharmaceutical composition of the present invention is a concentration of cells at which a therapeutic effect can be obtained for diseases in the case of administration to a patient or subject, as compared to a patient or subject without administration. A specific cell concentration can be appropriately determined depending on the administration form, an administration method, the purpose of use, and an age, a body weight, and symptoms of a patient or subject, and the like. A lower limit of the cell concentration of the pharmaceutical composition of the present invention is not particularly limited, but it is, for example, 1.0 × 10⁵ cells/mL or more, 1.0 × 10⁶ cells/mL or more, 1.2 × 10⁶ cells/mL or more, 1.4 × 10⁶ cells/mL or more, 1.6 × 10⁶ cells/mL or more, 1.8 × 10⁶ cells/mL or more, 2.0 × 10⁶ cells/mL or more, 3.0 × 10⁶ cells/mL or more, 4.0 × 10⁶ cells/mL or more, 5.0 × 10⁶ cells/mL or more, 6.0 × 10⁶ cells/mL or more, 7.0 × 10⁶ cells/mL or more, 8.0 × 10⁶ cells/mL or more, 9.0 × 10⁶ cells/mL or more, 9.5 × 10⁶ cells/mL or more, or 1.0 × 10⁷ cells/mL or more. An upper limit of the cell concentration of the pharmaceutical composition of the present invention is not particularly limited, but it is, for example, 1.0 × 10¹⁰ cells/mL or less, 1.0 × 10⁹ cells/mL or less, 8.0 × 10⁸ cells/mL or less, 6.0 × 10⁸ cells/mL or less, 4.0 × 10⁸ cells/mL or less, 2.0 × 10⁸ cells/mL or less, or 1.0 × 10⁸ cells/mL or less.

As one of embodiments of the pharmaceutical composition of the present invention, the pharmaceutical composition is a liquid medication and is preferably a liquid medication for injection. As the liquid medication for injection, for example, liquid preparations suitable for injection are known in PCT International Publication No. WO2011/043136, Japanese Unexamined Patent Application, First Publication No. 2013-256510, and the like.

The pharmaceutical composition of the present invention can also be the liquid medication for injection disclosed in the above documents. Furthermore, the above-mentioned liquid medication may be a suspension of cells, or may be a liquid preparation obtained by dispersing cells in a liquid medication. Furthermore, the morphology of cells contained in the liquid medication is not particularly limited, but it may be, for example, a single cell or may be a cell aggregate.

When the pharmaceutical composition of the present invention is a liquid medication for injection, a lower limit of a cell concentration of the liquid medication for injection is preferably 1.0 × 10⁶ cells/mL or more, 1.2 × 10⁶ cells/mL or more, 1.4 × 10⁶ cells/mL or more, 1.6 × 10⁶ cells/mL or more, 1.8 × 10⁶ cells/mL or more, 2.0 × 10⁶ cells/mL or more, 3.0 × 10⁶ cells/mL or more, 4.0 × 10⁶ cells/mL or more, 5.0 × 10⁶ cells/mL or more, 6.0 × 10⁶ cells/mL or more, 7.0 × 10⁶ cells/mL or more, 8.0 × 10⁶ cells/mL or more, 9.0 × 10⁶ cells/mL or more, 9.5 × 10⁶ cells/mL or more, or 1.0 × 10⁷ cells/mL or more from the viewpoint of enhancing therapeutic effects on diseases. Furthermore, an upper limit of a cell concentration of the liquid medication for injection is preferably 1.0 × 10⁹ cells/mL or less, 8.0 × 10⁸ cells/mL or less, 6.0 × 10⁸ cells/mL or less, 4.0 × 10⁸ cells/mL or less, 2.0 × 10⁸ cells/mL or less, or 1.0 × 10⁸ cells/mL or less from the viewpoint of facilitating preparation and administration of the liquid medication for injection.

Furthermore, according to another embodiment of the present invention, the pharmaceutical composition of the present invention may be a formulation for transplantation having a cell mass-like structure or a sheet-like structure. As the formulation for transplantation having a cell mass-like structure, for example, a formulation for transplantation which contains a cell aggregate obtained by adhering separated cells with an adhesive (for example, fibrinogen) is known in PCT International Publication No. WO2017/126549. Furthermore, as the formulation for transplantation having a sheet-like structure, for example, a cell sheet obtained by culture using a temperature-responsive culture dish (for example, UpCell (registered trademark) (manufactured by CellSeed Inc.)), a laminate of a sheet-shaped cell culture and a fibrin gel, a cell applied sheet obtained by applying a cell suspension onto a sheet-shaped substrate, and the like are known in PCT International Publication No. WO2006/080434, Japanese Unexamined Patent Application, First Publication No. 2016-52272, and the like. The pharmaceutical composition of the present invention can also be made into various kinds of formulations for transplantation having a cell mass-like structure or a sheet-like structure by using, for example, the method disclosed in the above documents.

Furthermore, according to still another embodiment of the present invention, the pharmaceutical composition of the present invention may be a gel formulation in which cells and an arbitrary gel are mixed. As the gel formulation, for example, a cell therapeutic agent containing a cell-hydrogel composition as an active ingredient is known in Published Japanese Translation No. 2017-529362 of the PCT International Publication. The pharmaceutical composition of the present invention can also be the gel formulation disclosed in the above document.

A method of administering the pharmaceutical composition of the present invention is not particularly limited. Examples thereof include subcutaneous injection, intracutaneous injection, intramuscular injection, intra-lymph node injection, intravenous injection, intra-arterial injection, intraperitoneal injection, intrathoracic injection, local direct injection, direct attachment, local direct transplantation, and the like. According to one aspect of the present invention, it is possible to fill a syringe with a liquid medication for injection and administer it, through an injection needle or a catheter, intravenously, intra-arterially, intramyocardially, into the joint cavity, into the hepatic artery, intramuscularly, epidurally, into the gingiva, intracerebroventricularly, subcutaneously, intracutaneously, intraperitoneally, and intraportally, but administration methods are not limited thereto. Regarding the method of administering the pharmaceutical composition, for example, intravenous injection, intravenous infusion, local direct injection, local direct transplantation, and the like are known in Japanese Unexamined Patent Application, First Publication No. 2015-61520; Onken JE, t al. American College of Gastroenterology Conference 2006 Las Vegas, NV, Abstract 121.; Garcia-Olmo D, et al. Dis Colon Rectum 2005, 48: 1416-23; and the like. The pharmaceutical composition of the present invention can also be administered by various methods disclosed in the above-mentioned documents.

An administration frequency of the pharmaceutical composition of the present invention is a frequency at which a therapeutic effect can be obtained for diseases in the case of administration to a patient or subject. A specific administration frequency can be appropriately determined depending on the administration form, an administration method, the purpose of use, and an age, a body weight, and symptoms of a patient or subject, and the like. The administration frequency is, for example, once every four weeks, once every three weeks, once every two weeks, once a week, twice a week, three times a week, four times a week, five times a week, six times a week, or seven times a week.

An administration period of the pharmaceutical composition of the present invention is a period in which a therapeutic effect can be obtained for diseases in the case of administration to a patient or subject. A specific administration period can be appropriately determined depending on the administration form, an administration method, the purpose of use, and an age, a body weight, and symptoms of a patient or subject, and the like. The administration period is, for example, 1 week, 2 weeks, 3 weeks, 4 weeks, 5 weeks, 6 weeks, 7 weeks, or 8 weeks.

A timing of administering the pharmaceutical composition of the present invention to a patient or subject is not particularly limited. Examples thereof include immediately after onset, within n days of onset (where n indicates an integer of 1 or more), immediately after diagnosis, within n days of diagnosis (where n indicates an integer of 1 or more), before remission, during remission, after remission, before relapse, during relapse, after relapse, and the like.

### [Examples]

The present invention will be described in more detail with reference to the following examples, but these are merely examples and do not limit the present invention.

### (Example 1)

### <Maintenance culture of pluripotent stem cells>

Regarding an iPS cell line RPChiPS771 (ReproCELL Inc.), undifferentiation maintenance culture was performed on SNL Feeder cells, which had been treated with Mitomycin-C (FUJIFILM Wako Pure Chemical Corporation), using an iPS cell medium (DMEM/HAM'S F12 (FUJIFILM Wako Pure Chemical Corporation) containing 20% Knockout Serum Replacement (KSR; GIBCO), IX Non-Essential Amino Acids (NEAA; FUJIFILM Wako Pure Chemical Corporation), 55 µmoL/L 2-Mercaptethanol (2-ME; GIBCO), 7.5 ng/mL recombinant human Fibroblast Growth Factor (FGF2; PeproTech, Inc.), and 0.5X Penicillin and Streptomycin (PS; FUJIFILM Wako Pure Chemical Corporation)). Alternatively, undifferentiation maintenance culture was performed on a plate coated with Vitronectin (GIBCO) using an ESSENTIAL 8^{™} medium (E8; GIBCO) containing IX Penicillin and Streptomycin and Amphotericin B (FUJIFILM Wako Pure Chemical Corporation). The culture was performed by adding Y27632 (FUJIFILM Wako Pure Chemical Corporation) so that a final concentration was 10 µM only at the time of seeding.

### <Process of raising temperature of medium to temperature at which pluripotent stem cells can proliferate>

An ESSENTIAL 8^{™} medium containing 500 mL and stored at a medium temperature of 4°C was injected into a 5 L culture tank until an amount of the medium reached 3.5 L. Next, the temperature of the medium was raised while controlling the temperature with a temperature sensor attached to the tank so that the temperature of the medium in the 5 L culture tank was around 37°C.

### <Suspension culture of pluripotent stem cells>

The iPS cells cultured in the above-described maintenance culture were treated with Accutase (Thermo Fisher Scientific K.K.) for about 5 minutes, detached, and dispersed into single cells. The dispersed cells were suspended in an ESSENTIAL 8^{™} medium containing BSA (FUJIFILM Wako Pure Chemical Corporation) at a final concentration of 5 mg/mL. Thereafter, a part thereof was collected and stained with trypan blue, and the number of the cells was measured. Based on measured values of the number of the cells, a cell suspension was prepared in the ESSENTIAL 8^{™} medium so that 2 × 10⁵ cells per mL were contained. After confirming that the temperature of the medium in the 5 L culture tank reached around 37°C, the cell suspension was seeded in the culture tank, and stirring culture was carried out for 5 days while maintaining an environment of 5% CO₂ and 37°C. After the culture, cell aggregates were suspended in TrypLE^{™} select (Life Technologies Corporation) and treated for 10 minutes in an environment of 5% CO₂ and 37°C. After the treatment, the cell aggregates were dispersed into single cells using a micropipette, and the number of the cells obtained after the culture was measured by trypan blue staining. As a result of the measurement of the number of the cells, 7 × 10⁹ pluripotent stem cells (cells/lot) could be obtained.

### (Comparative Example 1)

### <Process of repeatedly injecting medium, in which temperature of medium had been raised, into culture tank>

An ESSENTIAL 8^{™} medium containing 500 mL and stored at a medium temperature of 4°C was immersed in warm water at 42°C to raise the temperature of the medium in a medium container to around 37°C. Thereafter, 500 mL of the medium was injected into a 5 L culture tank which had been separately raised to around 37°C. The injection operation was repeated 6 times until an amount of the medium in the 5 L culture tank reached 3.5 L.

### <Suspension culture of pluripotent stem cells>

A cell suspension (2 × 10⁵ cells/mL) was prepared in the same manner as in Example 1. After confirming that the temperature of the medium in the 5 L culture tank was maintained at around 37°C, the cell suspension was seeded in the culture tank, and stirring culture was carried out for 5 days while maintaining an environment of 5% CO₂ and 37°C. As a result of measuring the number of the cells after the culture in the same manner as in Example 1, 1 × 10⁹ pluripotent stem cells (cells/lot) could be obtained, but it became clear that growth of pluripotent stem cells was significantly reduced as compared to Example 1.

### <Analysis of medium components>

In order to investigate the cause of the reduction in growth of the pluripotent stem cells, parts of the media in the 5 L culture tanks of Example 1 and Comparative Example 1 were collected, and a total amount of protein in each of the media was measured with a protein assay bicinchoninic acid kit (NACALAI TESQUE, INC.). As a result, it was found that the total amount of protein contained in the medium of Comparative Example 1 was reduced than that of Example 1. Furthermore, in order to identify a protein decreased in Comparative Example 1, parts of the media in the 5 L culture tanks of Example 1 and Comparative Example 1 were collected, and a content of FGF2 in each of the media was measured using FGF2 ELISA kits (BioLegend, Inc.). As a result, it was found that, although FGF2 could be detected in the medium of Example 1, FGF2 was lower than the detection sensitivity in the medium of Comparative Example 1. Based on these results, it was found that destabilization of the medium components is the cause of the reduction in growth of pluripotent stem cells in Comparative Example 1, and that specifically, the growth factors (particularly FGF2) were significantly reduced. Accordingly, it was confirmed that a large amount of pluripotent stem cells can be cultured by providing the process of raising the temperature of the medium in the culture tank to a temperature, at which the pluripotent stem cells can proliferate, before suspension culture of iPS cells as described in Example 1.

### <Undifferentiated state of pluripotent stem cells>

In order to examine the undifferentiated state of pluripotent stem cells, parts of the media in the 5 L culture tanks of Example 1 and Comparative Example 1 were collected, and each cell suspension was subjected to centrifugal separation at 300 g for 5 minutes. Thereafter, the supernatant was removed, and the cells were washed with phosphate buffered saline (PBS). Thereafter, the cells were fixed with 4% paraformaldehyde (PFA) at room temperature for 20 minutes, and thereafter washed with PBS three times. Permeation treatment was performed with cold methanol at -20°C overnight. After washing three times with PBS, the cells were blocked with 3% fetal bovine serum (FBS)/PBS and stained with fluorescently labeled anti-OCT4 (Cat. No. 653703, BioLegend, Inc.) at 4°C for 1 hour. After washing once with 3% FBS/PBS, pluripotent stem cells that had passed through a cell strainer were analyzed by FACSVerse. As a result of the analysis, a positive rate, for OCT4, of the pluripotent stem cells obtained by the method of Example 1 was 99%. Meanwhile, a positive rate, for OCT4, of the pluripotent stem cells obtained by the method of Comparative Example 1 was 55%. Based on these results, it was confirmed that an undifferentiated state of pluripotent stem cells can be efficiently maintained by providing the process of raising the temperature of the medium in the culture tank to a temperature, at which the pluripotent stem cells can proliferate, before suspension culture of the iPS cells as described in Example 1.

### (Comparative Example 2)

The influence on the growth of pluripotent stem cells in suspension culture, when the process of raising the temperature of the medium in the culture tank described in Example 1 to a temperature at which pluripotent stem cells can proliferate was not performed, was analyzed.

### <Suspension culture of pluripotent stem cells>

Maintenance culture and suspension culture of pluripotent stem cells were performed in the same manner as in Example 1 except that the process of raising the temperature of the medium in the culture tank described in Example 1 to a temperature at which pluripotent stem cells can proliferate was not performed. Specifically, an ESSENTIAL 8^{™} medium containing 500 mL and stored at a medium temperature of 4°C was injected into a 5 L culture tank until an amount of the medium reached 3.5 L. Next, after seeding the cell suspension of iPS cells in the culture tank, stirring culture was carried out in an environment of 5% CO₂ for 5 days while heating the temperature of the medium in the culture tank to around 37°C. As a result, only 9 × 10⁸ pluripotent stem cells (cells/lot) could be obtained.

### (Example 2)

### <Examination of culture scale>

Suspension culture of iPS cells was performed in the same manner as in Example 1 except that an amount of the culture in the culture tank was changed to 5 L, 10 L, 20 L, 30 L, 40 L, and 50 L. Table 1 shows the results of examining the number of the iPS cells after the completion of culture in each case. As a control test, the influence of the culture scale under the production conditions described in Comparative Example 1 was also tested.

**[Table 1]**

| | Amount of medium | | | | | |
|---|---|---|---|---|---|---|
| | 5 L | 10 L | 20 L | 30 L | 40 L | 50 L |
| Example 2 | 1 × 10¹⁰ cells/lot | 2 × 10¹⁰ cells/lot | 3.8 × 10¹⁰ cells/lot | 5.7 × 10¹⁰ cells/lot | 7.9 × 10¹⁰ cells/lot | 1 × 10¹¹ cells/lot |
| Control test (Comparative Example 1) | 2 × 10⁹ cells/lot | 3.8 × 10⁹ cells/lot | 4 × 10⁹ cells/lot | 4.5 × 10⁹ cells/lot | 5 × 10⁹ cells/lot | 4.8 × 10⁹ cells/lot |

As shown in the results in Table 1, it was confirmed that a large amount of pluripotent stem cells can be cultured by providing the process of raising the temperature of the medium in the culture tank to a temperature, at which the pluripotent stem cells can proliferate, before suspension culture of iPS cells. In particular, it was possible to efficiently perform culture as the amount of the medium increased, and the growth of pluripotent stem cells was improved 5 to 20 times as compared to the control test. Furthermore, by using the present invention, it was found that there is a correlation between the amount of the medium and the growth of pluripotent stem cells.

### (Example 3)

### <Influence of medium temperature before heating>

Suspension culture of iPS cells was performed in the same manner as in Example 1 except that the initial medium temperature in the culture tank was changed to -20°C, -15°C, -10°C, 0°C, 10°C, and 15°C, and the amount of the medium in the culture tank was changed to 10 L. Table 2 shows the results of examining the number of the iPS cells after the completion of culture in each case.

As a control test, the influence of the initial medium temperature under the production conditions described in Comparative Example 2 was also tested.

**[Table 2]**

| | Initial temperature of liquid medium | | | | |
|---|---|---|---|---|---|
| | -20°C | -10°C | 0°C | 10°C | 15°C |
| Example 3 | 1.5 × 10¹⁰ cells/lot | 1.8 × 10¹⁰ cells/lot | 1.7 × 10¹⁰ cells/lot | 1.9 × 10¹⁰ cells/lot | 1.9 × 10¹⁰ cells/lot |
| Control test (Comparative Example 2) | 0 cells/lot | 0 cells/lot | 1 × 10⁹ cells/lot | 7 × 10⁹ cells/lot | 1 × 10¹⁰ cells/lot |

As shown in the results in Table 2, it was confirmed that a large amount of pluripotent stem cells can be cultured by providing the process of raising the temperature of the medium in the culture tank to a temperature, at which the pluripotent stem cells can proliferate, before suspension culture of iPS cells. In particular, it was possible to efficiently perform culture as the temperature, which is a temperature of the liquid medium to be raised, increased, and the growth of pluripotent stem cells was improved 2 to 20 times as compared to the control test. Furthermore, by using the present invention, it was found that there is a correlation between the temperature change in the liquid medium and the growth of pluripotent stem cells.

### (Example 4)

### <Examination of conditions for adding growth factors>

64 mg/L of magnesium ascorbic acid-2-phosphate and 543 mg/L of sodium hydrogen carbonate were added to a DMEM/F12 medium stored at a medium temperature of 4°C, and the medium was injected into a 5 L culture tank until an amount of the medium reached 3.5 L. Next, the temperature of the medium was raised while controlling the temperature with a temperature sensor attached to the tank so that the temperature of the medium in the 5 L culture tank was around 37°C. Next, after confirming that the medium temperature in the 5 L tank was around 37°C, growth factors were added into the medium in the tank so that they were 1% ITS (insulin-transferrin-selenium; Life Technologies Corporation), 100 µg/L FGF2, and 2 µg/L TGF-β1. iPS cells cultured in the same manner as the undifferentiation maintenance described in Example 1 were seeded in the medium in the tank so that 2 × 10⁵ cells per mL were contained. Stirring culture was carried out for 5 days while maintaining an environment of 5% CO₂ and 37°C. After the culture, cell aggregates were suspended in TrypLE select and treated for 10 minutes in an environment of 5% CO₂ and 37°C. After the treatment, the cell aggregates were dispersed into single cells using a micropipette, and the number of the cells obtained after the culture was measured by trypan blue staining. As a result of the measurement of the number of the cells, 1.4 × 10¹⁰ pluripotent stem cells (cells/lot) could be obtained. Based on these results, it was found that the growth of pluripotent stem cells was significantly improved and was improved nearly twice as compared to the growth of Example 1, by adding the growth factors during the suspension culture process of pluripotent stem cells and not adding the growth factors during the process of raising the temperature of the medium in the culture tank to a temperature at which pluripotent stem cells could proliferate.

### (Example 5)

### <Induction of differentiation into somatic cells (pancreatic β cells)>

Regarding an iPS cell line RPChiPS771 (ReproCELL Inc.), undifferentiation maintenance culture was performed on SNL Feeder cells, which had been treated with Mitomycin-C (FUJIFILM Wako Pure Chemical Corporation), using an iPS cell medium (DMEM/HAM'S F12 (FUJIFILM Wako Pure Chemical Corporation) containing 20% Knockout Serum Replacement (KSR; GIBCO), IX Non-Essential Amino Acids (NEAA; FUJIFILM Wako Pure Chemical Corporation), 55 µmoL/L 2-Mercaptethanol (2-ME; GIBCO), 7.5 ng/mL FGF2, and 0.5X Penicillin and Streptomycin (PS; FUJIFILM Wako Pure Chemical Corporation)). An ESSENTIAL 8^{™} medium containing 500 mL and stored at a medium temperature of 4°C was injected into a 5 L culture tank until an amount of the medium reached 3.5 L. The temperature of the medium was raised while controlling the temperature with a temperature sensor attached to the tank so that the temperature of the medium in the 5 L culture tank was around 37°C. The iPS cells cultured in the above-described maintenance culture were treated with Accutase (Thermo Fisher Scientific K.K.) for about 5 minutes, detached, and dispersed into single cells. The dispersed cells were suspended in an ESSENTIAL 8^{™} medium containing BSA (FUJIFILM Wako Pure Chemical Corporation) at a final concentration of 5 mg/mL. Thereafter, a part thereof was collected and stained with trypan blue, and the number of the cells was measured. Based on measured values of the number of the cells, a cell suspension was prepared in the ESSENTIAL 8^{™} medium so that 2 × 10⁵ cells per mL were contained. After confirming that the temperature of the medium in the 5 L culture tank reached around 37°C, the cell suspension was seeded in the culture tank, and stirring culture was carried out for 5 days while maintaining an environment of 5% CO₂ and 37°C. For the first 2 days, in a state where a cell population of the iPS cells obtained in the above-described culture was adhered on a dish, the cell population was cultured in RPMI 1640 containing 0.5% Bovine Serum Albumin, 0.4 × PS, 1 mmol/L sodium pyruvate, 1 × NEAA, 80 ng/mL recombinant human activin A, 50 ng/mL FGF2, 20 ng/mL recombinant bone morphogenetic protein 4, and 3 µmol/L CHIR99021. On the 3rd day, CHIR99021 was removed from this medium, and culture was performed in a state where the cells were adhered on the dish. On the 4th day, in a medium into which 1% (volume/volume) KSR was further added, culture was performed for 1 day in a state where the cells were adhered on the dish to induce differentiation from pluripotent stem cells into endoderm cells. Next, culture was performed for 2 days in RPMI 1640 containing 0.5% BSA, 1 mmol/L sodium pyruvate, 1 × NEAA, 0.4 × PS, 50 ng/mL FGF2, 50 ng/mL recombinant human FGF7 (PeproTech, Inc.), 2% B27 supplement (GIBCO), 0.67 µmol/L EC23 (SANTA CRUZ), 1 µmol/L dorsomorphin (FUJIFILM Wako Pure Chemical Corporation), 10 µmol/L SB431542 (FUJIFILM Wako Pure Chemical Corporation), and 0.25 mol/L SANT1 (FUJIFILM Wako Pure Chemical Corporation) to induce differentiation from the endoderm cells into primitive gut tube (PGT) cells. Next, culture was performed for 4 days in DMEM-high glucose (FUJIFILM Wako Pure Chemical Corporation) containing 0.4 × PS, 1 × NEAA, 50 ng/mL FGF2, 2% B27, 0.67 µmol/L EC23, 1 µmol/L dorsomorphin, 10 µmol/L SB431542, and 0.25 µmol/L SANT1 to induce differentiation from the primitive gut tube (PGT) cells into posterior foregut (PFG) cells. Next, culture was performed for 3 days in DMEM-high glucose containing 0.4 × PS, 1 × NEAA, 50 ng/mL recombinant human FGF10 (PeproTech, Inc.), 2% B27, 0.5 µmol/L EC23, 1 µmol/L dorsomorphin, 0.25 µmol/L SANT1, 5 µmol/L Alk5 inhibitor II (BioVision Inc.), and 0.3 µmol/L indolactam V (ILV; Cayman Chemical Company) to induce differentiation from the posterior foregut (PFG) cells into pancreatic progenitor (PP) cells. Next, culture was performed for 3 days in Advanced-DMEM (GIBCO) containing 0.4 × PS, 2 mmol/L L-glutamine, 2% B27, 0.2 µmol/L EC23, 1 µmol/L dorsomorphin, 0.25 µmol/L SANT1, 5 µmol/L Alk5 inhibitor II, and 50 ng/mL Exendin 4 (Sigma-Aldrich) to induce differentiation from the pancreatic progenitor (PP) cells into endocrine progenitor (EP) cells. Next, culture was performed for 6 days in Advanced-DMEM containing 0.4 × PS, 2 mmol/L L-glutamine, 2% B27, 10 ng/mL BMP4, 10 ng/mL FGF2, 50 ng/mL recombinant human hepatocyte growth factor (HGF; PeproTech, Inc.), 50 ng/mL insulin-like growth factor 1 (IGF1; PeproTech, Inc.), 5 µmol/L Alk5 inhibitor II, 50 ng/mL Exendin 4, 5 mmol/L nicotinamide (Sigma-Aldrich), and 5 µmol/L forskolin (FUJIFILM Wako Pure Chemical Corporation) to induce differentiation from the endocrine progenitor (EP) cells into pancreatic β cells. Although the above-described method of inducing differentiation is one embodiment, it is possible to induce differentiation from pluripotent stem cells into pancreatic β cells, and thereby it is possible to produce somatic cells.

## Claims

1. A production method for pluripotent stem cells, the method comprising the following (a) and (b):
(a) a process of filling a culture container with a liquid medium and thereafter raising, the temperature of the liquid medium in the culture container to the temperature at which pluripotent stem cells can proliferate and
(b) a process of seeding pluripotent stem cells in the liquid medium in the culture container and culturing the pluripotent stem cells in suspension.

2. The production method according to Claim 1, wherein the temperature of the liquid medium is raised by at least 20°C in the process (a).

3. The production method according to Claim 1 or 2, wherein the temperature of the liquid medium in the culture container before raising the temperature is -20°C or higher and 15°C or lower in the process (a).

4. The production method according to any one of Claims 1 to 3, wherein the temperature at which the pluripotent stem cells can proliferate is 30°C or higher and 40°C or lower in the process (a).

5. The production method according to any one of Claims 1 to 4, wherein the temperature of the liquid medium is raised with stirring the liquid medium in the culture container in the process (a).

6. The production method according to any one of Claims 1 to 5, wherein a growth factor is not added in the process (a).

7. The production method according to any one of Claims 1 to 6, wherein a growth factor is added in the process (b).

8. The production method according to any one of Claims 1 to 7, wherein the liquid medium contains at least one from the group consisting of L-ascorbic acid, insulin, transferrin, selenium, and sodium hydrogen carbonate.

9. The production method according to any one of Claims 1 to 8, wherein the pluripotent stem cells are embryonic stem cells or induced pluripotent stem cells (iPS cells).

10. The production method according to Claim 6 or 7, wherein the growth factor is at least one selected from the group consisting of FGF2 and TGF-β1.

11. A production method for somatic cells, the method comprising the following (a) to (c):
(a) a process of filling a culture container with a liquid medium and thereafter raising, the temperature of the liquid medium in the culture container to the temperature at which pluripotent stem cells can proliferate;
(b) a process of seeding pluripotent stem cells in the liquid medium in the culture container and culturing the pluripotent stem cells in suspension; and
(c) a process of culturing the pluripotent stem cells obtained in the process (b) in the presence of a differentiation-inducing factor to induce differentiation.

12. The production method according to Claim 11, wherein the temperature of the liquid medium is raised by at least 20°C in the process (a).

13. The production method according to Claim 11 or 12, wherein the temperature of the liquid medium in the culture container before raising the temperature is -20°C or higher and 15°C or lower in the process (a).

14. The production method according to any one of Claims 11 to 13, wherein the temperature at which the pluripotent stem cells grow is 30°C or higher and 40°C or lower in the process (a).

15. The production method according to any one of Claims 11 to 14, wherein the temperature of the liquid medium is raised while stirring the liquid medium in the culture container in the process (a).

16. The production method according to any one of Claims 11 to 15, wherein a growth factor is not added in the process (a).

17. The production method according to any one of Claims 11 to 16, wherein a growth factor is added in the process (b).

18. The production method according to any one of Claims 11 to 17, wherein the somatic cells are at least one selected from the group consisting of cardiac muscle cells, skeletal muscle cells, nerve cells, megakaryocytes, hematopoietic stem cells, airway epithelial cells, germ cells, dendritic cells, eosinophils, mast cells, cartilage cells, T cells, erythropoietin-producing cells, intestinal epithelium, pancreatic cells, hepatocytes, alveolar epithelial cells, and kidney cells.

19. A pharmaceutical product composition comprising the somatic cells obtained by the production method according to any one of Claims 11 to 18 as an active ingredient.

20. The pharmaceutical product composition according to Claim 19, wherein the pharmaceutical product composition is a liquid medication for injection.

21. The pharmaceutical product composition according to Claim 19, wherein the pharmaceutical product composition is a formulation for transplantation having a cell mass-like structure or a sheet-like structure.
